**Europäisches Patentamt**

**European Patent Office**

**Office européen des brevets**

(11) Veröffentlichungsnummer: **0 045 079**
**B1**

# EUROPÄISCHE PATENTSCHRIFT

(45) Veröffentlichungstag der Patentschrift:
24.10.84

(51) Int. Cl.³: **C 07 D 213/82**

(21) Anmeldenummer: **81105902.1**

(22) Anmeldetag: **25.07.81**

(54) Verfahren zur Reinigung von Nicotinsäureamid I.

(30) Priorität: **30.07.80  DE 3028904**

(43) Veröffentlichungstag der Anmeldung:
**03.02.82 Patentblatt 82/5**

(45) Bekanntmachung des Hinweises auf die Patenterteilung:
**24.10.84 Patentblatt 84/43**

(84) Benannte Vertragsstaaten:
**AT BE CH DE FR GB IT LI NL SE**

(56) Entgegenhaltungen:
**DE - A - 2 517 053**
**DE - A - 2 517 054**
**DE - C - 828 247**
**US - A - 2 412 749**
**US - A - 2 471 518**
**US - A - 2 510 922**
**US - A - 2 544 157**

**Die Akte enthält technische Angaben, die nach dem Eingang der Anmeldung eingereicht wurden und die nicht in dieser Patentschrift enthalten sind.**

(73) Patentinhaber: **Degussa Aktiengesellschaft, Weissfrauenstrasse 9, D-6000 Frankfurt am Main 1 (DE)**

(72) Erfinder: **Beschke, Helmut, Greifenhagenstrasse 23, D-6450 Hanau 9 (DE)**
Erfinder: **Dahm, Franz-Ludwig, Dipl.-Ing., Kurmainzer Strasse 4, D-8755 Alzenau (DE)**
Erfinder: **Friedrich, Heinz, Dr. Dipl.-Chem., Grünaustrasse 4, D-6450 Hanau 9 (DE)**
Erfinder: **Prescher, Günter, Dr. Dipl.-Chem., Liesingstrasse 2, D-6450 Hanau 9 (DE)**

## Beschreibung

Die Erfindung betrifft ein Verfahren zur Gewinnung von reinem Nicotinsäureamid aus rohem, durch alkalische Hydrolyse von Nicotinsäurenitril erzeugtem Nicotinsäureamid durch Umkristallisation aus 2-Methylpropanol-(1). Insbesondere betrifft die Erfindung ein Verfahren zur Befreiung des Nicotinsäureamids von Verunreinigungen durch Nicotinsäure und Salzen der Nicotinsäure.

Nicotinsäureamid wird im allgemeinen durch Hydrolyse von Nicotinsäurenitril in alkalischem Medium erzeugt. Das bei diesen Herstellungsverfahren anfallende rohe Nicotinsäureamid enthält Verunreinigungen, insbesondere Nicotinsäure (im allgemeinen 0,3 bis 5,0%) und Salze der Nicotinsäure (im allgemeinen 1,5 bis 2,5%). Diese Verunreinigungen stören bei der Weiterverwendung des Nicotinsäureamids, namentlich auf pharmazeutischem Gebiet, insbesondere wenn ihr Anteil 0,1% übersteigt.

Es ist bekannt, rohes Nicotinsäureamid mit Hilfe von Ionenaustauschern zu reinigen (GB-A-879 551, US-A-3 678 060). Diese Verfahren sind aufwendig und ergeben zudem ein hinreichend reines Nicotinsäureamid nur unter erheblichen Ausbeuteeinbussen. Es ist auch bekannt, rohes Nicotinsäureamid durch Umkristallisation zu reinigen. Als Lösungsmittel dienen hierbei Aceton (US-A-2 471 518), Propanol-(2), oder Butylacetat in Gegenwart von Kohle (DE-C-828 247), Äthylacetat (J. Am. Chem. Soc. 65 (1943), 2256 bis 2257), Äthanol in Gegenwart von Kohle (J.Am. Chem. Soc. 70 (1948), 3945), Dioxan oder Petroläther (US-A-2 412 749) oder Benzol (DK-A-87 228). Nachteilig ist bei diesen Verfahren, dass zur Erzielung eines genügend reinen Nicotinsäureamids mehrfache Umkristallisation erforderlich ist und nur eine mässige Ausbeute an reinem Nicotinsäureamid erzielt wird.

Es ist auch bekannt, ein durch alkalische Hydrolyse von Nicotinsäurenitril erzeugtes Nicotinsäureamid durch Umkristallisation aus 2-Methylpropanol-(1) zu reinigen (DE-A-2517054). Auch bei diesem Verfahren ist die Ausbeute an reinem Nicotinsäureamid unbefriedigend.

Es ist nun ein Verfahren zur Gewinnung von reinem Nicotinsäureamid aus rohem, durch alkalische Hydrolyse von Nicotinsäurenitril erzeugtem Nicotinsäureamid durch Umkristallisation aus 2-Methylpropanol-(1) gefunden worden, das dadurch gekennzeichnet ist, dass das 2-Methylpropanol-(1) 10–18 Gew.-% Wasser enthält und die Umkristallisation bei einem pH-Wert zwischen 7 und 10 vorgenommen wird. Bei diesem Verfahren wird bei nur einer einzigen Umkristallisation mit günstiger Ausbeute ein hervorragend reines Nicotinsäureamid gewonnen.

Das erfindungsgemässe Verfahren eignet sich zur Reinigung rohen Nicotinsäureamids, wie es aus den Umsetzungsgemischen gewonnen wird, die beiden üblichen Verfahren zur Herstellung von Nicotinsäureamid anfallen, nämlich bei der Hydrolyse von Nicotinsäurenitril in alkalischem Medium. Mit Vorteil wird das Verfahren zur Reinigung des nach dem Verfahren gemäss der DE-A-2517054 erzeugten Nicotinsäureamids verwendet.

Zur Ausführung des erfindungsgemässen Verfahrens wird das rohe Nicotinsäureamid aus 2-Methylpropanol-(1) umkristallisiert, das 10 bis 18 Gewichtsprozent Wasser enthält. Vorteilhaft ist es, ein mit Wasser gesättigtes 2-Methylpropanol-(1) zu verwenden.

Das rohe Nicotinsäureamid wird für die Umkristallisation auf einen pH-Wert zwischen 7 und 10 eingestellt, und zwar mit Vorteil in der zur Umkristallisation auf 80 bis 100 °C erwärmten Lösung des Nicotinsäureamids in dem Wasser enthaltenden 2-Methylpropanol-(1). Bevorzugt wird ein pH-Wert von 7,2 bis 9,8, insbesondere von 7,5 bis 9,5, gewählt. Die Einstellung des pH-Wertes erfolgt, den Erfordernissen entsprechend, durch Zusatz von Säure, beispielsweise von Essigsäure, oder von Alkali, beispielsweise von Natriumhydroxid.

Die nach der Umkristallisation und Abscheidung des reinen Nicotinsäureamids verbleibende Mutterlauge wird mit Vorteil für weitere Umkristallisationen verwendet. Bei der kontinuierlichen Ausgestaltung des Verfahrens wird die Mutterlauge wiederholt im Kreislauf geführt, jedoch wird zur Vermeidung einer unerwünschten Anreicherung der Verunreinigungen in der Mutterlauge von Zeit zu Zeit oder zweckmässigerweise bei jedem Kreislauf ein, dem Grad der Verunreinigung des rohen Nicotinsäurenitrils entsprechender, Anteil der Mutterlauge entfernt. Es ist vorteilhaft, diesen Anteil der Mutterlauge aufzuarbeiten und beispielsweise entweder das 2-Methylpropanol-(1) und das Nicotinsäureamid durch Destillation abzutrennen und in den Kreislauf zurückzuführen, oder vorzugsweise die Verunreinigungen durch Behandlung mit Ionenaustauschern zu entfernen und die verbleibende gereinigte Mutterlauge wiederzuverwenden.

In den folgenden Beispielen bedeutet % stets Gewichtsprozent.

Beispiel 1

Es wurden 250 g rohes Nicotinsäureamid, das 2,3% Natriumnicotinat und 0,7% Nicotinsäure enthielt, mit 260 ml 2-Methylpropanol-(1) und 37 ml Wasser vermischt. Die Mischung wurde auf Siedetemperatur erhitzt und das Nicotinsäureamid hierbei aufgelöst. Die Lösung wurde auf 80 °C abgekühlt. Der pH-Wert des Gemisches war 6,6. Durch tropfenweise Zugabe von 5,3 ml 10prozentiger wässriger Natriumhydroxid-Lösung wurde der pH-Wert auf 7,9 erhöht. Schliesslich wurde langsam auf 10 °C abgekühlt. Das auskristallisierte Nicotinsäureamid wurde abgesaugt, dreimal mit je 50 ml wasserfreiem 2-Methylpropanol-(1) gewaschen und getrocknet. Die Ausbeute betrug 180 g, entsprechend 74%, bezogen auf das mit der rohen Substanz eingesetzte Nicotinsäureamid. In dem gewonnenen Nicotinsäureamid war Nicotinsäure nicht nachweisbar. Der Natriumgehalt betrug 0,001%.

Beispiel 2

Es wurde wie nach Beispiel 1 verfahren, jedoch wurde durch Zugabe von 5,1 ml der Natriumhydroxid-Lösung der pH-Wert auf 7,3 eingestellt. Die Ausbeute betrug 174 g, entsprechend 72%. Das Produkt wies die gleiche Reinheit wie das nach Beispiel 1 gewonnene Produkt auf.

Beispiel 3

Es wurden 320 g einer Mutterlauge, die bei einer vorangegangenen Umkristallisation angefallen war, vorgelegt. In dieser wurden 180 g rohes Nicotinsäureamid, das 2,3% Natriumnicotinat und 0,7% Nicotinsäure enthielt, bei Siedetemperatur gelöst. Durch Zugabe von 3,9 ml 10prozentiger wässriger Natriumhydroxid-Lösung wurde der pH-Wert auf 7,9 eingestellt. Dann wurde auf 10 °C abgekühlt. Das auskristallisierte Nicotinsäureamid wurde abgesaugt, dreimal mit je 50 ml wasserfreiem 2-Methylpropanol-(1) gewaschen und getrocknet. Die Ausbeute betrug 171 g, entsprechend 98%, bezogen auf das mit der rohen Substanz eingesetzte Nicotinsäureamid. Das Produkt wies die gleiche Reinheit wie das nach Beispiel 1 gewonnene Produkt auf.

Beispiel 4

Es wurde die Mutterlauge verwendet, die bei der Umkristallisation gemäss Beispiel 3 angefallen war. Von der Gesamtmenge von 320 g wurden 195 g abgetrennt, eingedampft und bei 145 °C und 1 mbar destilliert. Neben dem Lösungsmittel wurden hierbei 7 g reines Nicotinsäureamid gewonnen. Die restlichen 225 g Mutterlauge wurden nach Zusatz von 78 g 2-Methylpropanol-(1) und 6 g Wasser für eine weitere Umkristallisation verwendet. Hierzu wurden 109 g rohes Nicotinsäureamid eingesetzt, das 2,3% Natriumnicotinat und 0,7% Nicotinsäure enthielt. Im übrigen wurde wie nach Beispiel 3 verfahren. Die Ausbeute betrug 97 g, entsprechend 91%, bezogen auf das mit der rohen Substanz eingesetzte Nicotinsäureamid. Das Produkt wies die gleiche Reinheit wie das nach Beispiel 1 gewonnene Produkt auf. Die Mutterlauge wurde jeweils nach Abtrennung eines Anteils von 95 g und Zusatz von 78 g 2-Methylpropanol-(1) und 6 g Wasser für weitere gleiche Umkristallisationen verwendet.

Beispiel 5

Es wurde wie nach Beispiel 3 verfahren, jedoch wurden 10-fache Mengen eingesetzt. Bei der Umkristallisation fielen 3300 g Mutterlauge an. Von dieser wurde ein Anteil von 1100 g abgetrennt und gereinigt. Hierzu wurde dieser Flüssigkeitsanteil zunächst über eine Säule eines stark sauren Kationenaustauschers gegeben. Die Durchflussgeschwindigkeit betrug 3,5 Liter Flüssigkeit je Liter Austauscher und Stunde. Der Austauscher wurde bis zu einer Kapazität von 1,62 val je Liter Austauscher genutzt und ergab eine Senkung des Gehalts an Natriumionen in der Flüssigkeit auf unter 1 ppm, bezogen auf den Gehalt an Nicotinsäureamid in der Flüssigkeit. Die Flüssigkeit wurde dann über einen Anionenaustauscher geleitet. Dieser wurde bis zu einer Kapazität von 0,38 val je Liter Austauscher genutzt und bewirkte, dass der Gehalt an Nicotinationen auf unter 0,02%, bezogen auf den Gehalt an Nicotinsäureamid in der Flüssigkeit, gesenkt wurde. Der so gereinigte Mutterlaugenanteil wurde mit der übrigen Mutterlauge vereinigt und für eine weitere Umkristallisation verwendet. Hierbei wurde wie nach Beispiel 3 verfahren, jedoch wurden die Substanzen in 10-facher Menge eingesetzt. Das Produkt wies die gleiche Reinheit wie das nach Beispiel 1 gewonnene Produkt auf. Die Ausbeute betrug 93%. Der Kationenaustauscher wurde durch Behandlung mit verdünnter wässriger Chlorwasserstoffsäure und der Anionenaustauscher durch Behandlung mit verdünnter wässriger Natriumhydroxid-Lösung regeneriert. Dabei fielen 47 g Natriumnicotinat an.

**Patentanspruch**

Verfahren zur Gewinnung von reinem Nicotinsäureamid aus rohem, durch alkalische Hydrolyse von Nicotinsäurenitril erzeugtem Nicotinsäureamid durch Umkristallisation aus 2-Methylpropanol-(1) dadurch gekennzeichnet, dass das 2-Methylpropanol-(1) 10–18 Gew.-% Wasser enthält und die Umkristallisation bei einem pH-Wert zwischen 7 und 10 vorgenommen wird.

**Revendication**

Procédé d'obtention d'amide nicotinique pur à partir du produit brut obtenu par hydrolyse alcaline de nitrile nicotinique, par recristallisation dans le 2-méthylpropanol-(1), procédé caractérisé en ce que le 2-méthylpropanol-(1) contient 10 à 18% en poids d'eau et que la recristallisation est effectuée à un pH compris entre 7 et 10.

**Claim**

A process for the recovery of pure nicotinic acid amide from crude nicotinic acid amide, which is produced by the alkaline hydrolysis of nicotinic acid nitrile, by recrystallisation from 2-methylpropan-1-ol, characterised in that the 2-methyl-propan-1-ol contains from 10 to 18%, by weight, of water and recrystallisation is carried out at a pH value ranging from 7 to 10.